# EUROPÄISCHE PATENTSCHRIFT

(11) **EP 0 934 430 B1**
(45) Veröffentlichungstag und Bekanntmachung des Hinweises auf die Patenterteilung: **12.12.2001**
(21) Anmeldenummer: 97912018.5
(22) Anmeldetag: 25.09.1997
(51) Int. Cl.: C12Q 1/37

(54) **VERFAHREN ZUM DIAGNOSTISCHEN NACHWEIS VON APOPTOSE IN ZELLEN**
METHOD FOR APOPTOSIS DIAGNOSIS IN CELLS
PROCEDE DE DIAGNOSTIC D'APOPTOSE DANS DES CELLULES

(30) Priorität: 25.09.1996 DE 19639450
(43) Veröffentlichungstag der Anmeldung: 11.08.1999
(73) Patentinhaber: Deutsches Krebsforschungszentrum, 69120 Heidelberg (DE)
(72) Erfinder: DEBATIN, Klaus-Michael, D-69120 Heidelberg (DE); LOS, Marek, D-69121 Heidelberg (DE); HUG, Hubert, D-69118 Heidelberg (DE)
(74) Vertreter: Schüssler, Andrea, Dr.
(86) Internationale Anmeldenummer: DE9702204
(87) Internationale Veröffentlichungsnummer: WO9813517

(56) Entgegenhaltungen:
- EP-A- 0 428 000
- WO-A-93/25694
- WO-A-95/34576
- WO-A-96/13607
- V. GAGLIARDINI ET AL.: "Prevention of vertebrate neuronal death by the crmA gene." SCIENCE., Bd. 263, 11.Februar 1994, LANCASTER, PA US, Seiten 826-828, XP002056344
- E. D. MATAYOSHI ET AL.: "Novel fluorogenic substrates for assaying retroviral proteases by resonance energy transfer." SCIENCE., Bd. 247, 23.Februar 1990, LANCASTER, PA US, Seiten 954-958, XP002056345 in der Anmeldung erwähnt

## Beschreibung

Die vorliegende Erfindung betrifft ein Verfahren zum diagnostischen Nachweis von Apoptose in Zellen und einen hierfür verwendbaren Kit.

Alle derzeitigen Methoden zum Nachweis von Apoptose detektieren relativ späte Ereignisse. Darüber hinaus sind diese Methoden nur wenig geeignet, Apoptose ex vivo nach Apoptoseinduktion nachzuweisen, da apoptotische Zellen in vivo Veränderungen aufweisen, die mit einer Modifikation in der Zellmembran einhergehen, so daß apoptotische Zellen sehr rasch in vivo vom retikuloendothelialen System eliminiert werden. Das führt dazu, daß apoptotische Zellen für eine ex vivo Detektion dann nicht mehr zur Verfügung stehen. Dies ist ein großer Nachteil, da der Nachweis der Initiierung oder Suppression von Apoptose in vivo immer größere diagnostische Bedeutung erlangt, einerseits um den Zytostatika-induzierten Zelltod zu verfolgen, andererseits um HIV-Erkrankungen oder Neurodegeneration, die gesteigerten Zellumsatz und Zelltod durch verstärkte Apoptose hervorrufen, zu erkennen.

Die Aufgabe der vorliegenden Erfindung besteht deshalb darin, ein Verfahren bereitzustellen, mit dem zuverlässig das Ausmaß an Apoptose in Zellen abgeschätzt werden kann.

Gelöst wird diese Aufgabe durch ein Verfahren gemäß Patentanspruch 1 sowie einen Kit gemäß Patentanspruch 3 gelöst. Vorteilhafte Ausgestaltungen ergeben sich aus den Unteransprüchen.

Der molekulare Mechanismus durch welchen Antikrebs-Medikamente Apoptose in Zielzellen auslösen ist noch relativ unbekannt. Von den Erfindern wurde in jüngster Zeit gefunden, daß die durch Zytostatika in Tumorzellen induzierte Apoptose die Aktivierung von Proteasen der ICE/Ced3-Familie erfordert. Nach Gabe des Medikaments wurde ein starkes Ansteigen der ICE-ähnlichen Aktivität gefunden, welche dem Zelltod vorausgeht. Die gefundenen Daten geben zu dem Schluß Anlaß, daß alle Apoptose auslösenden Ereignisse einen gemeinsamen Signalweg betätigen, an dem letztendlich der Zelltod steht. Für die Effektorphase bei intrazellulären Signalwegen, die zu Apoptose führen, ist die Aktivierung der ICE/Ced3-Familie essentiell. Die Aktivierung von ICE-Proteasen geht dem morphologisch messbaren apoptotischen Zelltod und dem Auftreten von DNA-Strangbrüchen um mehrere Stunden voraus und ist somit ein früher Parameter der Apoptose in ansonsten noch intakten Zellen. Somit stellt die Aktivierung von ICE/Ced3-Proteasen ein frühes Ereignis in Zellen dar, in denen irreversibel das Apoptoseprogramm induziert wurde.

Die Erfindung beruht nun auf der Erkenntnis der Erfinder, daß über die verfolgbare Aktivierung der ICE/Ced3-Proteasen auf molekularer Ebene frühe Ereignisse, die mit der Induktion von intrazellulären Apoptoseprogrammen assoziiert sind, detektiert werden können. Vorzugsweise werden ex vivo Proben von Patienten vermessen, die unter einer apoptoseinduzierenden oder apoptoseinhibierenden Therapie stehen. Mit dem erfindungsgemäßen Verfahren ist es deshalb möglich, die Bestimmung der Wirkung von Therapien anhand der Induktion bzw. Inhibierung von Apoptose ex vivo oder in vivo an intakten Zellen zuverlässig vorzunehmen.

Zur Durchführung der quantitativen Messung der Induktion des Apoptoseprogramms wird die Aktivierung der ICE/Ced3-Proteasen mittels einer bildgebenden (optischen) Methode, vorzugsweise einer Flow-cytometrischen Methode, detektiert. Zu diesem Zweck werden intakte Zellen nach Permeabilisierung mit Substraten für ICE/Ced3-Proteasen beladen. Die Permeabilisierung kann beispielsweise durch Zugabe von Wasser zu den Zellen (osmotischer Schock) oder durch Einwirkung von Digitonin bzw. anderen Permeabilisatoren erfolgen. Bevorzugt findet die Permeabilisierung soweit statt, daß 10% der Zellen danach als lebend im forward/side-scatter erscheinen. Geeignete Substrate für die ICE/Ced3-Proteasen weisen mindestens eine Aspartylgruppe auf. Die Protease-Substrate sind vorzugsweise zur Durchführung einer Flow-cytometrischen Messsung fluorogen. Geeignete Substrate sind in "Matayoshi et al., Science 247, S. 954-958 (1990)" beschrieben. Vorzugsweise wird ein fluorogenes Substrat verwendet, bei dem 5-[(2-Aminoethyl)amino]naphthalin-1-sulfonsäure (EDANS) den fluorogenen Donor und 4-(4-Dimethylaminophenylazo)-benzoesäure (DABCYL) den quenchenden Akzeptor darstellt, wobei Donor und Akzeptor miteinander mittels eines Spacers verbunden sind. Ganz besonders bevorzugt werden DABCYL-YVADAPK-EDANS oder DAB-CYL-DEVDAPK-EDANS verwendet. Ferner können auch Cumarin-verwandte Substrate, wie DEVD-NMA, verwendet werden. Ebenso eignen sich Rhodamin 110-Derivate, bei denen Aminogruppen mit verschiedenen einzelnen Aminosäuren oder kurzen Caspase-Substratpeptiden substituiert sind. Die intrazelluläre Proteaseaktivität wird optisch, vorzugsweise mittels eines Flow-Cytometers, als Fluoreszenz des gespaltenen Subtrats gemessen. Gleichzeitig ermöglicht die Methode die Identifikation der Zellen über Oberflächenmarker in der Simultanmessung, d.h. bei verschiedenen Zellen kann exakt bestimmt werden, welche Zellen noch leben und welche abgestorben sind.

Die Vorteile des erfindungsgemäßen Verfahrens sind, daß es schnell und unkompliziert durchführbar ist und eine sensitive Detektion ermöglicht. Der Apoptosenachweis ist mehrere Stunden, bevor morphologisch faßbare Veränderungen in den Zellen auftreten, möglich und wird nicht durch die Beseitigung apoptotischer Zellen durch das retikuloendotheliale System in vivo beeinflußt. Das erfindungsgemäße VerfahrenJäßt sich auch mittels eines Kits durchführen. Dieser enthält ein vorstehendes Substrat für Proteasen der ICE/Ced3-Familie, wobei das Substrat insbesondere fluorogen ist. Ganz besonders bevorzugt sind Substrate, wie DABCYL-YVADAPK-EDANS, DABCYL-DEVDAPK-EDANS, DEVD-NMA oder Rhodamin 110-Derivate, bei denen Aminogruppen mit verschiedenen einzelnen Aminosäuren oder kurzen Caspase-Substratpeptiden substituiert sind.

Die vorliegende Erfindung wird nun anhand der Figuren weiter beschreiben, von denen zeigen:
- Fig. 1: 2D-Plots der gemessenen ICE/Ced3-Aktivität (x-Achse) gegen die gemessene Autofluoreszenz bei 660 nm (y-Achse)
- Fig. 2: Auftrag der gemessenen ICE/Ced3-Aktivität in einem Histogramm
Kontrollzellen : weiß
nach anti-APO-1-Behandlung: schwarz
- Fig. 3: Messung der durch Doxorubicin-induzierten ICE/Ced3-Aktivität und Zelltod (Höchst 33342/Propidiumjodid) in CEM-Zellen in einer Zeitkinetik
Die ICE/Ced3-Aktivität (Quadrate) kann 2-4 Stunden vor den ersten DNA-Veränderungen, die apoptotischen Zelltod zeigen (Rauten), detektiert werden.
- Fig. 4: Messung der Fluoreszenzintensität (Caspasen-Aktivität) und Zellzahl bei Jurkat-Zellen nach Behandlung mit anti-APO-1

Die Erfindung wird nun weiter anhand der folgenden Beispiele beschrieben.

### BEISPIEL 1

### 1. Variante

CEM-Zellen, die für 1 Stunde mit einem für CD95-spezifischen Antikörper (anti-APO-1; 1*µ*g/ml) behandelt worden sind (vgl. Oehm, A. et al., The Journal of Biological Chemistry, Band 267, Nr. 15 (1992), S. 10709-10715), werden in Zellkulturmedium RPM11640 + 10% FCS kultiviert. Es werden 10 *µ*l ICE/Ced3-Substrat DABCYL-YVADAP-EDANS (Stocklösung 100 mM in DMSO; aufbewahrt bei - 70°C) zugegeben. Es erfolgt die Zugabe von Wasser (gleiches Volumen wie das Zellkulturmedium) zur Permeabilisierung der Zellen. Die Mischung wird für 5 Minuten bei 37°C inkubiert bevor eiskaltes 5x PBS in 50% FCS (0,25 Volumen des Anfangsvolumens) zugegeben wird, um die Osmolarität auf den normalen Wert zu bringen. Die Mischung wird auf Eis aufbewahrt und innerhalb von 2 Stunden im Flow-Cytometer vermessen. Die Anregungswellenlänge des verwendeten Subtrats beträgt ∼ 360 nm und die Emission wird bei 488 nm gemessen.

Als Kontrolle werden unbehandelte CEM-Zellen dem gleichen Prozedere unterworfen.

### 2. Variante

CEM-Zellen, die für 2 Stunden mit einem für CD95-spezifischen agonistischen Antikörper (anti-APO-1; 1*µ*g/ml) behandelt worden sind (vgl. Oehm, A. et al., The Journal of Biological Chemistry, Band 267, Nr. 15 (1992), S. 10709-10715), werden in Zellkulturmedium (RPMI1640 + 10% FCS) kultiviert. Es werden 10 *µ*l ICE/Ced3-Substrat DABCYL-YVADAP-EDANS (Stocklösung 100 mM in DMSO; aufbewahrt bei -70°C) und Digitonin in einer Endkonzentration von 0,002-0,005% (bevorzugte Konzentration: 10% der Zellen erscheinen nach Permeabilisierung als lebend im forward/side-scatter) zugegeben. Die Mischung wird 5 Minuten bei 37°C inkubiert bevor ein gleiches Volumen eiskaltes Zellkulturmedium oder PBS zugegeben wird, um das Digitonin zu verdünnen. Die Mischung wird auf Eis aufbewahrt und innerhalb von 2 Stunden im Flow-Cytometer vermessen. Die Anregungswellenlänge des verwendeten Subtrats beträgt ∼ 360 nm und die Emission wird bei 488 nm gemessen.

Als Kontrolle werden unbehandelte CEM-Zellen dem gleichen Prozedere unterworfen.

Es ist anzumerken, daß die Variante 2 effektiver ist, allerdings liefern die nach Variante 1 behandelten Zellen einen niedrigeren Hintergrund.

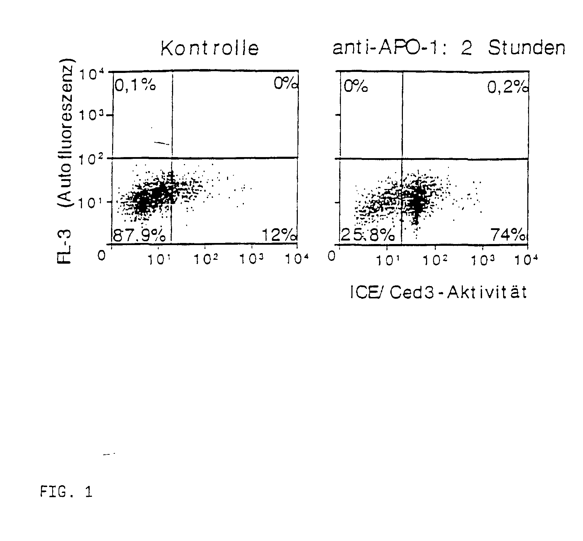

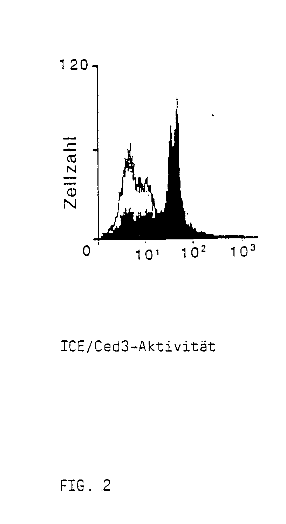

Die Ergebnisse sind in den Figuren 1 und 2 gezeigt. Daraus ist klar ersichtlich, daß die behandelten Zellen eine deutliche ICE/Ced3-Aktivität zeigen, während die Aktivität bei den unbehandelten Zellen vernachlässigbar gering ist.

### BEISPIEL 2

CEM-Zellen, die mit Doxorubicin (1*µ*g/ml) behandelt worden sind, werden in Zellkulturmedium (RPMI1640 + 10% FCS) kultiviert. Es werden 10 *µ*l ICE/Ced3-Substrat DABCYL-YVADAP-EDANS (Stocklösung 100 mM in DMSO; aufbewahrt bei -70°C) und Digitonin in einer Endkonzentration von 0,002-0,005% (bevorzugte Konzentration: 10% der Zellen erscheinen nach Permeabilisierung als lebend im forward/side-scatter) zugegeben. Die Mischung wird 5 Minuten bei 37°C inkubiert bevor ein gleiches Volumen eiskaltes Zellkulturmedium oder PBS zugegeben wird, um das Digitonin zu verdünnen. Die Mischung wird auf Eis aufbewahrt und innerhalb von 2 Stunden im Flow-Cytometer vermessen. Die Anregungswellenlänge des verwendeten Subtrats beträgt ∼360 nm und die Emission wird bei 488 nm gemessen.

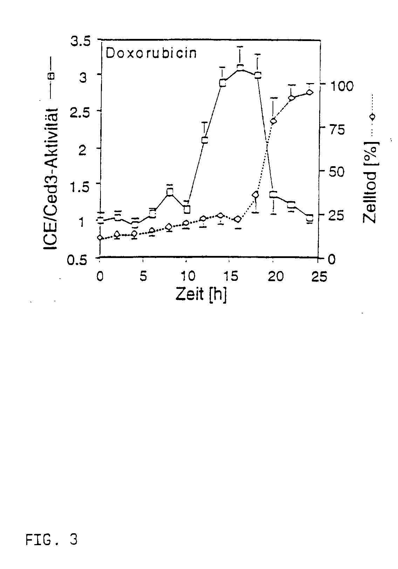

Die aufgenommene Zeitkinetik ist in Fig. 3 gezeigt. Daraus ist ersichtlich, daß die ICE/Ced3-Aktivität lange vor Eintritt des Zelltodes nachweisbar ist und ein sicheres Indiz für begonnene Apoptose liefert.

### BEISPIEL 3

Jurkat-Zellen werden 1,5 h mit einem für CD95-spezifischen Antikörper (anti-APO-1) behandelt. Sie werden dann behandelt, wie in der Variante 1 von Beispiel 1 beschrieben. Als ICE/Ced3-Substrat wird das Caspasen-Substrat D₂Rhodamin bis zu einer Endkonzentration von 50*µ*M zugegeben. Die Caspase-Aktivität wird in einem Flow-Cytometer (FACScan, Becton Dickinson) gemessen. Die Anregungswellenlänge beträgt 488 nm. Die Fluoreszenz wird im FL1-Photomultiplier gemessen. Die punktierte Linie zeigt die Kontrollzellen, die nicht mit dem anti-CD95-Antikörper behandelt wurden. Die durchgezogene Linie zeigt die Fluoreszenzverschiebung nach Antikörper-Behandlung. Durch Propidiumiodid-Färbung läßt sich erst 3 h nach Antikörper-Behandlung eine Zunahme von toten Zellen nachweisen.

Als Kontrolle werden unbehandelte Jurkat-Zellen dem gleichen Prozedere unterworfen.

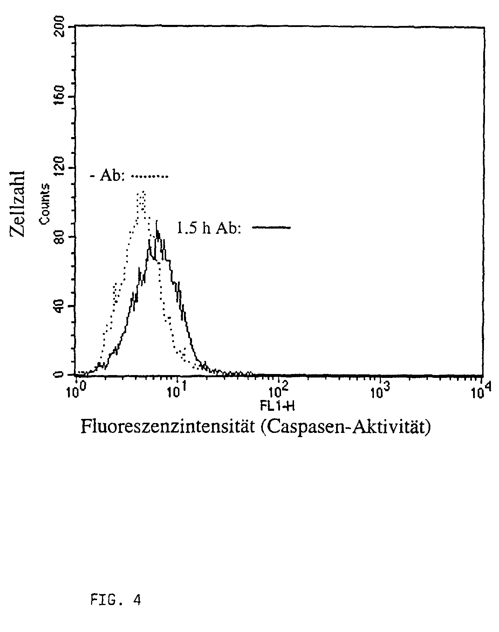

Das Ergebnis ist in Fig. 4 gezeigt. Daraus ist ersichtlich, daß bei den behandelten Zellen gegenüber den unbehandelten Zellen eine Fluoreszenzverschiebung auftritt, was die unterschiedliche ICE/Ced3-Aktivität wiedergibt.

## Patentansprüche

1. Verfahren zum Nachweis von Apoptose in Zellen, wobei die Zellen ex vivo mit einem fluorogenen Substrat für Proteasen der ICE/Ced3-Familie, das mindestens eine Aspartylgruppen enthält, beladen werden und die Proteaseaktivität mittels Flow-Cytometrie als Fluoreszenz des gespaltenen Substrats gemessen wird.

2. Verfahren nach Anspruch 1, **dadurch gekennzeichnet, daß** das Substrat DABCYL-YVADAPK-EDANS, DABCYL-DEVDAPK-EDANS, DEVD-NMA oder ein Rhodamin 110-Derivat, bei dem Aminogruppen mit verschiedenen einzelnen Aminosäuren oder kurzen Caspase-Substratpeptiden substituiert sind, ist.

3. Kit zur Durchführung des Verfahrens nach Anspruch 1 oder 2 enthaltend ein fluorogenes Substrat für Proteasen der ICE/Ced3-Familie, das mindestens eine Aspartylgruppe enthält.

4. Kit nach Anspruch 3, **dadurch gekennzeichnet, daß** das Substrat DABCYL-YVADAPK-EDANS, DABCYL-DEVDAPK-EDANS, DEVD-NMA oder ein Rhodamin 110-Derivat, bei dem Aminogruppen mit verschiedenen einzelnen Aminosäuren oder kurzen Caspase-Substratpeptiden substituiert sind, ist.

## Claims

1. A method for apoptosis diagnosis in cells, wherein the cells are loaded ex vivo with a fluorogenic substrate for ICE/Ced3 family proteases, containing at least one aspartyl group and the protease acitivity of the cleaved substrate is measured as fluorescence by flow-cytometry.

2. The method according to claim 1, **characterized in that** the substrate is DABCYL-YVADAPK-EDANS, DABCYL-DEVDAPK-EDANS, DEVD-NMA or a rhodamine 110 derivative, in which amino groups are substituted with various individual amino acids or short caspase substrate peptides.

3. A kit for carrying out the method according to claim 1 or 2, containing a fluorogenic substrate for ICE-Ced3 family proteases, which contains at least one aspartyl group.

4. A kit according to claim 3, **characterized in that** the substrate is DABCYL-YVADAPK-EDANS, DABCYL-DEVDAPK-EDANS, DEVD-NMA or a rhodamine 110 derivative, in which amino groups are substituted with various individual amino acids or short caspase substrate peptides.

## Revendications

1. Procédé de détection d'apoptose dans des cellules, dans lequel on charge les cellules ex vivo avec un substrat fluorogène pour protéases de la famille ICE/Ced3, qui contient au moins un groupe aspartyle, et on mesure l'activité de la protéase par cytométrie de flux en fonction de la fluorescence du substrat séparé.

2. Procédé selon la revendication 1, **caractérisé en ce que** le substrat est DABCYL-YVADAPK-EDANS, DABCYL-DEVDAPK-EDANS, DEVD-NMA ou un dérivé 110 de rhodamine, dans lequel des groupes amino sont substitués par différents aminoacides individuels ou par de courts peptides substrats de la caspase.

3. Kit pour la mise en oeuvre du procédé selon la revendication 1 ou 2, contenant un substrat fluorogène pour protéases de la famille ICE/Ced3, qui contient au moins un groupe aspartyle.

4. Kit selon la revendication 3, **caractérisé en ce que** le substrat est DABCYL-YVADAPK-EDANS, DABCYL-DEVDAPK-EDANS, DEVD-NMA ou un dérivé 110 de rhodamine, dans lequel des groupes amino sont substitués par différents aminoacides individuels ou par de courts peptides substrats de la caspase.
